(19) Europäisches Patentamt
European Patent Office
Office européen des brevets

(11) EP 0 618 920 B1

(12) EUROPÄISCHE PATENTSCHRIFT

(45) Veröffentlichungstag und Bekanntmachung des Hinweises auf die Patenterteilung:
**14.08.1996 Patentblatt 1996/33**

(21) Anmeldenummer: **93900042.8**

(22) Anmeldetag: **14.12.1992**

(51) Int Cl.6: **C07F 9/38**, A61K 31/66, C07F 9/40, C07F 9/572, C07F 9/6506, C07F 9/58, C07F 9/553

(86) Internationale Anmeldenummer:
**PCT/EP92/02890**

(87) Internationale Veröffentlichungsnummer:
**WO 93/12122 (24.06.1993 Gazette 1993/15)**

(54) **NEUE PHOSPHONOBERNSTEINSÄUREDERIVATE, VERFAHREN ZU DEREN HERSTELLUNG UND DIESE VERBINDUNGEN ENTHALTENDE ARZNEIMITTEL**

NOVEL PHOSPHONO-SUCCINIC ACID DERIVATIVES, PROCESS FOR PRODUCING THEM AND MEDICAMENTS CONTAINING THESE COMPOUNDS

NOUVEAUX DERIVES DE L'ACIDE PHOSPHONOSUCCINIQUE, LEUR PROCEDE DE FABRICATION ET MEDICAMENTS RENFERMANT CES COMPOSES

(84) Benannte Vertragsstaaten:
**AT BE CH DE DK ES FR GB GR IE IT LI LU NL PT SE**

(30) Priorität: **19.12.1991 DE 4141928**

(43) Veröffentlichungstag der Anmeldung:
**12.10.1994 Patentblatt 1994/41**

(73) Patentinhaber: **BOEHRINGER MANNHEIM GMBH**
**D-68298 Mannheim (DE)**

(72) Erfinder:
- **TSAKLAKIDIS, Christos**
  **D-6940 Weinheim (DE)**
- **BOSIES, Elmar**
  **D-6940 Weinheim (DE)**
- **ESSWEIN, Angelika**
  **D-7700 Singen (DE)**
- **BAUSS, Frieder**
  **D-6715 Lambsheim (DE)**

(74) Vertreter: **Weber, Manfred, Dr. et al**
**c/o Boehringer Mannheim GmbH,**
**Patentabteilung,**
**Sandhoferstrasse 116**
**68298 Mannheim (DE)**

(56) Entgegenhaltungen:
**EP-A- 0 186 405** **FR-A- 2 253 527**

- **PHOSPHORUS AND SULFUR Vol. 13, Nr. 1, 1982 Seiten 85-95 R. BURGADA ' Addition des fonctions NH et P(O)H sur la double liaison des vinyl spirophosphoranes ' cited in the Application.**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 618 920 B1

Printed by Jouve, 75001 PARIS (FR)

## Beschreibung

Die vorliegende Erfindung betrifft neue Phosphonobernsteinsäurederivate, Verfahren zu deren Herstellung sowie Arzneimittel, die diese Substanzen enthalten.

In EP 0 186 405 sind pharmazeutische Zusammensetzungen beschrieben, die geminale Diphosphonate enthalten, sowie deren Verwendung bei abnormalen Calcium und Phosphatmetabolismus.

In FR-2253527 sind ebenfalls pharmazeutische Zusammensetzungen beschrieben, die verschiedene Phosphonoalkylbernsteinsäurederivate enthalten.

In Phosphorus and Sulfur 13, 85 (1982) ist die Synthese des 3-Dimethylamino-2-dimethylphosphonobernsteinsäuredimethylester beschrieben, eine pharmakologische Wirkung dieser Verbindung ist jedoch nicht bekannt.

Es wurde nun gefunden, daß analoge Phosphonobernsteinsäure-derivate eine ausgezeichnete Wirkung auf den Calciumstoffwechsel zeigen und damit zur breiten Behandlung von Calcium-stoffwechselstörungen geeignet sind. Sie lassen sich vor allem sehr gut dort einsetzen, wo der Knochenauf- und abbau gestört ist, d.h. sie sind geeignet zur Behandlung von Erkrankungen des Skelettsystems wie z.B. Osteoporose, Morbus Paget, Morbus Bechterew u.a..

Aufgrund dieser Eigenschaften finden sie aber auch Verwendung in der Therapie der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose.

Gegenstand der vorliegenden Erfindung sind Verbindungen der allgemeinen Formel I,

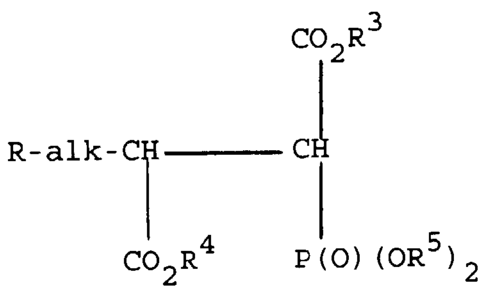

(I)

in der

R eine gegebenenfalls substituierte Aminogruppe der allgemeinen Formel $-NR_1R_2$, wobei $R_1$ und $R_2$ unabhängig voneinander jeweils Wasserstoff, niederes Alkyl, niederes Alkenyl oder niederes Alkinyl bedeutet, oder R einen gesättigten, ungesättigten oder aromatischen heterocyclischen Ring darstellt, der gegebenenfalls ein- oder zweifach durch niederes Alkyl, Chlor oder Brom substituiert sein kann,

alk einen Valenzstrich, eine Methylen-, eine gesättigte oder ungesättigte, geradkettige oder verzweigte Alkylenkette mit 2-6 Kohlenstoffatomen bedeutet, und

$R^3$, $R^4$, $R^5$ jeweils unabhängig voneinander Wasserstoff, niederes Alkyl oder Benzyl bedeuten,

sowie deren pharmakologisch unbedenkliche Salze,

wobei für den Fall, daß $R^3=R^4=R^5=CH_3$ und alk einen Valenzstrich bedeutet, R nicht die Dimethylaminogruppe sein darf.

Niederes Alkyl soll in allen Fällen eine geradkettige oder verzweigte $C_1$-$C_6$-Alkylgruppe wie z.B. Methyl, Ethyl, Propyl, Isopropyl, Butyl, Isobutyl, Pentyl oder Hexyl, insbesondere Methyl, Ethyl, Propyl, Isobutyl und Pentyl darstellen.

Niederes Alkenyl bedeutet ungesättigte Reste mit 3-6 Kohlenstoffatomen wie z.B. Allyl, But-2-enyl, Hexa-2,4-dienyl, vor allem Allyl.

Niederes Alkinyl soll ungesättigte Reste mit 3-6 Kohlenstoffatomen darstellen wie z.B. Propargyl, But-3-inyl, Hex-5-inyl, insbesondere aber Propargyl.

Falls der Rest R einen gesättigten heterocyclischen Ring bedeutet, handelt es sich um 3-8-gliedrige Ringe, die noch ein oder zwei weitere Heteroatome enthalten können, wie den Aziridin-, Azetidin-, Pyrrolidin-, Piperidin, Azepin-, Morpholin- oder den Thiomorpholinring, insbesondere den Pyrrolidin-, Azepin- und den Morpholinring.

Falls R einen ungesättigten heterocyclischen Ring bedeutet, handelt es sich in der Regel um den Imidazolinring.

Falls R einen hetero-aromatischen Ring darstellt, handelt es sich um fünf- oder sechsgliedrige Ringe, wie den Pyridin-, Pyrimidin-, Pyrazin-, Imidazol-, insbesondere den Pyridin- und Imidazolring.

Die heterocyclischen Ringe können gegebenenfalls ein oder zweifach durch $C_1$-$C_6$-Alkylqruppen, vorzugsweise die Methyl-, Ethyl- oder Isopropylgruppe, sowie durch Chlor oder Brom substituiert sein.

alk stellt im Falle der gesättigten oder ungesättigten, geradkettigen oder verzweigten Alkylenkette Reste wie z.B. Methylen, Ethylen, Propylen, Butylen, 2-Methylpropylen, Pentylen, 1,1-Dimethyl-propylen, 2,3-Dimethyl-propylen, 2,2-Dimethylpropylen, 2-Methyl-butylen, Hexylen, 2,3-Dimethyl-butylen, 2-Methyl-pentylen, 2-Butenylen, 2-Butinylen, insbesondere Methylen, Ethylen, Propylen, Butylen, 2-Methyl-propylen, Pentylen, Hexylen und 2-Butenylen dar.

Verbindungen der allgemeinen Formel I

enthalten mindestens zwei asymmetrische Kohlenstoffatome, daher sind auch optisch aktive Verbindungen der allgemeinen Formel I Gegenstand der vorliegenden Anmeldung.

Verbindungen der allgemeinen Formel I werden nach an sich bekannten Verfahren hergestellt, vorzugsweise dadurch, daß man

a) Carbonsäurederivate der allgemeinen Formel II,

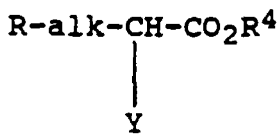

(II)

in der R, alk und $R^4$ die oben angegebenen Bedeutungen haben und Y eine Abgangsgruppe wie z.B. Hal oder O-$SO_2$-Z bedeuten, wobei Hal Chlorid, Bromid oder Jodid und Z Methyl, Phenyl, p-Methylphenyl oder p-Nitrophenyl sein sollen, mit einem Phosphonoessigsäureester der allgemeinen Formel III,

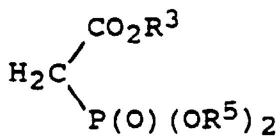

(III)

in der $R^3$ und $R^5$ die oben angegebene Bedeutung besitzen, umsetzt, wobei für den Fall, daß R eine primäre oder sekundäre Aminogruppe bedeutet, diese in geschützter Form etwa als Acylamino- oder Phthaloylimidogruppe vorliegen muß, und gegebenenfalls die entstandenen Ester teilweise oder vollständig zu den entsprechenden Säuren der allgemeinen Formel I verseift, oder

b) Verbindungen der allgemeinen Formel IV,

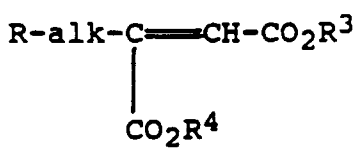

(IV)

in der R, alk, $R^3$ und $R^4$ die oben angegebenen Bedeutungen haben, mit einem Dialkylphosphit der allgemeinen Formel V,

$$H\text{-}P(O)\,(OR^5)_2 \qquad (V),$$

in der $R^5$ die oben angegebener Bedeutungen hat, umsetzt und gegebenenfalls die entstandenen Ester teilweise oder vollständig zu den entsprechenden Säuren der allgemeinen Formel I verseift, oder

c) eine Verbindung der allgemeinen Formel VI oder VII,

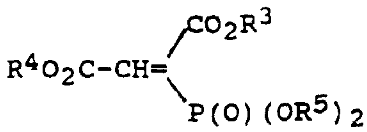

,

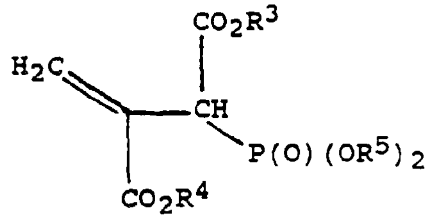

VI

VII

in der $R^3$, $R^4$ und $R^5$ die oben angegebenen Bedeutungen besitzen, in an sich bekannter Weise mit einer Verbindung der allgemeinen Formel VIII,

R-alk-M (VIII)

in der R die oben genannten Bedeutungen besitzt, und M Wasserstoff oder ein Alkali- bzw. Erdalkalimetall bedeutet, zur Reaktion bringt und gegebenenfalls die entstandenen Ester teilweise oder vollständig zu den entsprechenden Säuren der allgemeinen Formel I verseift und gewünschtenfalls in pharmakologisch verträgliche Salze überführt.

Verbindungen der allgemeinen Formel II werden so hergestellt, daß man für den Fall, daß Y = Hal bedeutet, eine Verbindung der allgemeinen Formel IX,

$R\text{-alk-}CH_2\text{-}CO_2R^4$ (IX)

in der R, alk und $R^4$ die oben genannten Bedeutungen besitzen, nach literaturbekannten Verfahren halogeniert, oder für den Fall, daß Y in Formel II die $O\text{-}SO_2\text{-}Z$-Gruppe bedeutet, die Hydroxylgruppe einer Verbindung der allgemeinen Formel (X),

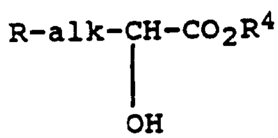

(X)

in der R, alk und $R^4$ die oben genannten Bedeutungen besitzen, in den entsprechenden Sulfonsäureester überführt.

Verbindungen der allgemeinen Formel III sind teilweise käuflich erwerblich (Aldrich-Chemie GmbH u. Co.KG) und werden in Spezialfällen nach bekannten Verfahren durch Umsetzung eines Halogenessigsäurederivats der allgemeinen Formel XI,

$Hal\text{-}CH_2\text{-}CO_2R^3$ (XI)

in der Hal und $R^3$ die oben angegebenen Bedeutungen besitzen, mit einem Triphosphit der allgemeinen Formel XII,

$P(OR^5)_3$ (XII)

in der $R^5$ die oben angegebenen Bedeutungen besitzt, hergestellt.

Verbindungen der allgemeinen Formel IV werden dadurch hergestellt, daß man

1. eine Verbindung der allgemeinen Formel XIII,

R-H (XIII)

in der R die oben genannten Bedeutungen besitzt, mit einer Verbindung der allgemeinen Formel XIV,

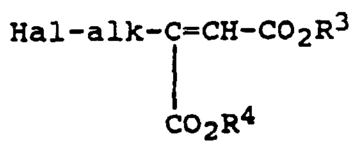

(XIV)

in der Hal, alk, $R^3$ und $R^4$ die oben genannten Bedeutungen besitzen, alkyliert, oder

2. eine Verbindung der allgemeinen Formel XV,

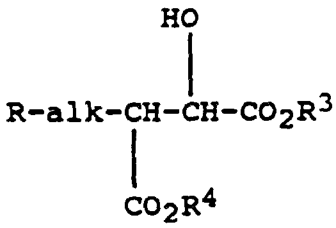

(XV),

in der R, alk, $R^3$ und $R^4$ die oben genannten Bedeutungen besitzen, nach bekannten Verfahren dehydratisiert.

Verbindungen der allgemeinen Formel V sind käuflich erhältlich (Aldrich Co.).

Verbindungen der allgemeinen Formel VI werden hergestellt, indem man eine Verbindung der allgemeinen Formel V mit einem Acetylendicarbonsäureester der allgemeinen Formel XVI,

$$R^4O_2C\text{-}C\text{-}C\text{-}CO_2R^3 \qquad \text{(XVI)}$$

in der $R^3$ und $R^4$ die oben angegebenen Bedeutungen haben, zur Reaktion bringt.

Verbindungen der allgemeinen Formel VII werden in an sich bekannter Weise hergestellt, indem man eine Verbindung der allgemeinen Formel XVII,

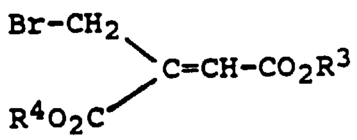

(XVII)

in der $R^3$ und $R^4$ die oben genannten Bedeutungen besitzen, mit einer Verbindung der allgemeinen Formel XII umsetzt.

Verbindungen der allgemeinen Formel VIII werden für den Fall, daß M nicht Wasserstoff bedeutet, entsprechend den Literaturverfahren metalliert.

Verbindungen der allgemeinen Formel IX werden nach bekannten Verfahren durch Alkylierung einer Verbindung der allgemeinen Formel XIII mit einer Verbindung der allgemeinen Formel XVIII,

$$\text{Hal-alk-}CH_2\text{-}CO_2R^4 \qquad \text{(XVIII)}$$

in der Hal, alk und $R^4$ die oben genannten Bedeutungen besitzen, erhalten.

Verbindungen der allgemeinen Formel X lassen sich nach Literaturverfahren durch Oxidation der entsprechenden Verbindungen der allgemeinen Formel IX erhalten.

Verbindungen der allgemeinen Formel XIV können in an sich bekannter Weise hergestellt werden, indem man eine Verbindung der allgemeinen Formel XIX,

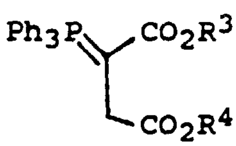

(XIX)

in der $R^3$ und $R^4$ die oben genannten Bedeutungen besitzen, mit einer Verbindung der allgemeinen Formel XX,

$$\text{Hal-alk-Hal} \qquad \text{(XX)}$$

in der Hal und alk die oben genannten Bedeutungen haben, umsetzt.

Verbindungen der allgemeinen Formel XV werden in an sich bekannter Weise durch Reaktion einer Verbindung der allgemeinen Formel IX mit einer Verbindung der allgemeinen Formel XXI,

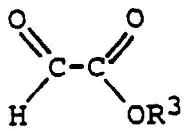

(XXI)

in der $R^3$ die oben genannten Bedeutungen besitzt, hergestellt.

Verbindungen der allgemeinen Formel XVII erhält man nach bekannten Methoden durch allylische Bromierung einer Verbindung der allgemeinen Formel XXII,

$$R^4O_2C-C(CH_3)=CH-CO_2R^3$$

(XXII),

in der $R^3$ und $R^4$ die oben genannten Bedeutungen besitzen.

Die Halogenierung einer Verbindung der allgemeinen Formel IX erfolgt durch ihre Umsetzung mit molekularem Halogen (Chlor, Brom, Iod) vorzugsweise Brom ohne Lösungsmittel oder in einem inerten Lösungsmittel wie Methylenchlorid, Chloroform oder Tetrachlorkohlenstoff, vorzugsweise Tetrachlorkohlenstoff und unter Zusatz von rotem Phosphor, Phosphortrichlorid oder Phosphortribromid und bei einer Temperatur zwischen Raumtemperatur und 100°C, vorzugsweise bei 90°C (K. Stoh, Chem. Pharm. Bull. 34, 2078 (1986); H. J. Ziegler, Synthesis 1969, 39)). Weiter lassen sich Verbindungen der allgemeinen Formel IX dadurch halogenieren, daß man sie in einem aprotischen Lösungsmittel wie Tetrahydrofuran und bei niedriger Temperatur, bevorzugt bei -78°C mit einem Lithiumamid wie Lithiumdiisopropylamid metalliert und anschließend die in -Stellung metallierte Verbindungen der allgemeinen Formel IX mit Brom, Iod, Tetrachlorkohlenstoff oder Tetrabromkohlenstoff (M. Hesse, Helv. Chim. Acta 72, 847 (1989); R.T. Arnold, J. Org. Chem. 43, 3687 (1978) bzw. mit N-Chlor- oder N-Bromsuccinimid (W. Oppolzer, Tetrahedron Lett. 26, 5037 (1985)) umsetzt.

Die Überführung der Hydroxylgruppe einer Verbindung der allgemeinen Formel X in einen Sulfonsäureester erfolgt nach üblichen Verfahren, wie z.B. durch die Kondensation mit einem Sulfonsäurechlorid, wie Methan-, Benzol-, p-Toluol- oder p-Nitrobenzolsulfonsäurechlorid, vorzugsweise Methan- oder p-Toluolsulfonsäurechlorid, in einem inerten Lösungsmittel wie Methylenchlorid, Tetrahyrofuran oder Diethylether, vorzugsweise Methylenchlorid unter Verwendung einer Hilfsbase wie Trimethyl-oder Triethylamin oder Pyridin, vorzugsweise Triethylamin und bei einer Temperatur zwischen 0°C und Raumtemperatur.

Zur Darstellung von Verbindungen der allgemeinen Formel XIX siehe R. Eyjolfsson, Acta, Chem. Scand., 3075 (1970).

Die Umsetzung einer Verbindung der allgemeinen Formel II mit einer Verbindung der allgemeinen Formel III erfolgt in der Regel in einem aprotischen Lösungsmittel wie Toluol, Tetrahydrofuran, Diethylether oder Dimethylformamid, vorzugsweise Dimethylformamid, oder Tetrahydrofuran unter Verwendung einer starken Base wie Kaliumhydrid, Natriumhydrid Lithiumdiisopropylamid oder Lithiumhexanethyldisilylamid, vorzugsweise Natriumhydrid oder Lithiumdiisopropylamid und bei Temperaturen zwischen -78°C und 90°C bevorzugt jedoch zwischen -10°C und Raumtemperatur.

Die Umsetzung einer Verbindung der allgemeinen Formel IV mit einer Verbindung der allgemeinen Formel V findet unter den Bedingungen der Michaeladdition, in einem Lösungsmittel wie Methanol, Ethanol, Toluol, Tetrahydrofuran, Diethylether, oder Dimethylformamid, vorzugsweise Methanol, Tetrahydrofuran oder Dimethylformamid ohne weitere Zusätze oder unter Verwendung einer Base wie Natrium- oder Kaliummethylat oder -ethylat, Natriumhydrid, Kaliumhydrid oder Lithiumdiisopropylamid, vorzugsweise Natriummethylat, Natriumhydrid oder Lithiumdiisopropylamid und bei Temperaturen zwischen -78°C und 90°C bevorzugt jedoch zwischen -10°C und Raumtemperatur statt.

Die Reaktion zwischen einer Verbindung der allgemeinen Formel VI bzw. VII mit einer Verbindung der allgemeinen Formel VIII führt man in der Regel unter den Bedingungen der Michaeladdition in einem Lösungsmittel wie Methanol, Ethanol, Toluol, Tetrahydrofuran, Diethylether oder Dimethylformamid, vorzugsweise Methanol, Tetrahydrofuran oder Dimethylformamid ohne weitere Zusätze oder unter Verwendung einer Base wie Natriumhydrid, Kaliumhydrid, Lithiumdiisopropylamid, Butyllithium, Ethylmagnesiumbromid, und gegebenenfalls Kupfersalz, wie Kupferchlorid- oder bromid zur Bildung des entsprechenden Cuprates einer Verbindung der allgemeinen Formel VIII (vgl. G.H. Posner, Tetrahedron Letters 37, 3215 (1977)), und bei Temperaturen zwischen -78°C und 90°C bevorzugt zwischen -78°C und Raumtemperatur durch.

Die Reaktion zwischen einer Verbindung der allgemeinen Formel XI mit einer Verbindung der allgemeinen Formel XII erfolgt in der Regel ohne Lösungsmittel bei Temperaturen zwischen Raumtemperatur und 150°C, vorzugsweise bei 130°C mit einer Reaktionszeit zwischen 30 min und 30 Stunden, vorzugsweise 18 Stunden.

Die Alkylierung einer Verbindung der allgemeinen Formel XIII mit einer Verbindung der allgemeinen Formel XIV

oder einer Verbindung der allgemeinen Formel XVIII führt man in der Regel in einem Lösungsmittel wie Methanol, Ethanol, Propanol, Tetrahydrofuran, Diethylether oder Dimethylformamid, vorzugsweise Methanol, Tetrahydrofuran oder Dimethylformamid, ohne Hilfsbasen oder unter Zugabe einer Base wie Kaliumcarbonat, Natriummethylat, Natrium- oder Kaliumhydrid, Lithiumdiisopropylamid, Butyllithium oder Phenyllithium vorzugsweise Natriumhydrid, Kaliumcarbonat, Butyllithium, oder Phenyllithium und bei einer Temperatur zwischen -78°C und der Rückflußtemperatur des verwendeten Lösungsmittels, vorzugsweise zwischen -78°C und 50°C, durch. Die Dehydratisierung einer Verbindung der allgemeinen Formel XV findet gewöhnlich in einem Lösungsmittel wie Benzol, Toluol, Xylol, Chloroform oder Methylenchlorid, vorzugsweise Toluol oder Methylenchlorid unter Zusatz eines Dehydratisierungsmittels wie Schwefelsäure, Phosphorsäure, p-Toluolsulfonsäure, vorzugsweise p-Toluolsulfonsäure und bei einer Temperatur zwischen Raumtemperatur und Rückflußtemperatur des verwendeten Lösungsmittels, vorzugsweise bei 100°C, statt. Zur Umsetzung einer Verbindung V mit einer Verbindung XVI siehe R. Burgada, Phosphorus and Sulfur 13, 85 (1982).

Die Reaktion einer Verbindung XII mit einer Verbindung XVII erfolgt in der Regel ohne Lösungsmittel bei Temperaturen zwischen 50°C und 180°C vorzugsweise bei 150°C.

Die Umsetzung einer Verbindung der Formel XIX mit einer Verbindung der Formel XX führt man in der Regel in einem inerten Lösungsmittel wie Tetrahydrofuran unter Verwendung einer Base wie Lithiumdiisopropylamid und bei einer Temperatur von -78°C durch (M. P. Cooke, Tetrahedron Lett. 22 381 (1981)).

Die Kondensation eines Carbonsäureesters der allgemeinen Formel IX mit einem Aldehyd der Formel XXI führt man gewöhnlich in einem Lösungsmittel wie Methanol, Ethanol, Tetrahydrofuran, Diethylether oder Dimethylformamid, vorzugsweise Methanol oder Tetrahydrofuran in Gegenwart eines basischen Kondensationsmittels wie Natriummethylat oder -ethylat, Kalium-tert.-butylat, Natriumhydrid oder Lithiumdiisopropylamid, vorzugsweise Natriummethylat, Kalium-tert.-butylat oder Lithiumdiisopropylamid und bei Temperaturen zwischen -78°C und 60°C, vorzugsweise zwischen -78°C und Raumtemperatur durch.

Zur allylischen Bromierung von 2-Methylfumar- oder maleinsäure und ihren Derivaten siehe J. Org. Chem. 34, 1228 (1969). Die Oxidation einer Verbindung der allgemeinen Formel IX zu einer Verbindung der allgemeinen Formel X führt man in der Regel in einem Lösungsmittel wie Tetrahydrofuran durch Zugabe einer Base wie Lithiumdiisopropylamid oder Lithium-N-Isopropyl-N-Cyclohexylamid unter Verwendung eines Oxidationsmittels wie einem Oxaziridin-Derivat, Molibdänperoxid oder Luftsauerstoff und bei Temperaturen zwischen -78°C und Raumtemperatur, vorzugsweise bei 50°C durch (C. Tamm. Tetrahedron Lett. 26, 203 (1985); F. A. Davis J. Org. Chem. 51, 2402 (1986); C. Winotai Synth. Commun. 18, 2141 (1988)).

Die freie Phosphonsäure-Gruppe in Verbindungen der allgemeinen Formel I kann durch Erhitzen mit Orthoameisensäuretrialkylestern in den entsprechenden Dialkylester überführt werden. Die Hydrolyse einer Phosphonsäureester-Gruppe in Verbindungen der allgemeinen Formel I zu der entsprechenden freien Phosphonsäure-Gruppe erfolgt in der Regel ohne Lösungsmittel oder in einem inerten Lösungsmittel wie Methylenchlorid durch ein Trimethylsilylhalogenid, wie Trimethylsilylbromid oder -jodid und bei einer Temperatur zwischen -50° und Raumtemperatur, vorzugsweise bei 0°C.

Die Veresterung der freien Carbonsäure-Gruppen in Verbindungen der allgemeinen Formel I erfolgt nach literaturbekannten Verfahren durch Erhitzen einer Verbindung der allgemeinen Formel I, in der $R^3$ und/oder $R^4$ Wasserstoff bedeutet, mit einem in dem herzustellenden Carbonsäureester enthaltenden Alkohol unter Zusatz eines sauren Katalysators wie Salzsäure, Schwefelsäure oder p-Toluolsulfonsäure, vorzugsweise Schwefelsäure. Die Verseifung einer Carbonsäureester-Gruppe in Verbindungen der allgemeinen Formel I führt man nach üblichen Verfahren durch, indem man einen Carbonsäureester der allgemeinen Formel I in Wasser oder in Gemischen aus Wasser, Tetrahydrofuran, Dioxan, Methanol oder Ethanol, vorzugsweise in einem Wasser/Tetrahydrofurangemisch mit einem Hydroxid wie Natrium-, Kalium- oder Lithiumhydroxid, vorzugsweise Natrium- oder Lithiumhydroxid und bei Temperaturen zwischen Raumtemperatur und 80°C, vorzugsweise bei Raumtemperatur, behandelt.

Die Schutzgruppe einer primären oder sekundären Aminogruppe in Verbindungen der allgemeinen Formel I läßt sich dadurch entfernen, daß man nach üblichen Verfahren eine Verbindung der allgemeinen Formel I, in der R eine Acylamino- oder Phthaloylimidogruppe bedeutet, mit wäßrigen Mineralsäuren bzw. -basen, wie Salzsäure oder Schwefelsäure bzw. Natron- oder Kalilauge behandelt, oder sie mit Hydrazin oder Hydroxylamin umsetzt.

Phosphon- und Carbonsäureester-Gruppen in Verbindungen der allgemeinen Formel I lassen sich weiterhin durch Kochen mit Chlor- oder Bromwasserstoffsäure verseifen. Liegen in Verbindungen der allgemeine Formel I Benzylester vor, so lassen sie sich hydrogenolytisch in die entsprechenden freien Phosphon-bzw. Carbonsäuren überführen.

Als pharmakologisch verträgliche Salze werden vor allem Mono-bzw. Dialkali- oder Ammoniumsalze verwendet, die man in üblicher Weise z. B. durch Titration der Verbindungen mit anorganischen oder organischen Basen wie z.B. Natrium- oder Kaliumhydrogencarbonat, Natronlauge, Kalilauge, wässrigem Ammoniak oder Aminen wie. z.B. Trimethyl- oder Triethylamin hergestellt werden.

Die Salze werden in der Regel durch Umfällen aus Wasser/Aceton gereinigt.

Die erfindungsgemäßen neuen Substanzen der Formel I und ihre Salze können in flüssiger oder fester Form enteral oder parenteral appliziert werden. Hierbei kommen alle üblichen Applikationsformen infrage, beispielsweise

Tabletten, Kapseln, Dragees, Sirupe, Lösungen, Suspensionen etc.. Als Injektionsmedium kommt vorzugsweise Wasser zur Anwendung, welches die bei Injektionslösungen üblichen Zusätze wie Stabilisierungsmittel, Lösungsvermittler und Puffer enthält.

Derartige Zusäzte sind z.B. Tartrat- und Citrat-Puffer, Ethanol, Komplexbildner (wie Ethylendiamintetraessigäsure und deren nichttoxische Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z.B. Stärke, Lactose, Mannit, Methylcellulose, Talkum, hochdisperse Kieselsäuren, höhermolekulare Fettsäuren (wie Sterinsäure), Gelatine, Agar-Agar, Calciumphosphat, Magnesiumstearat, tierische und pflanzliche Fette, feste hochmolekulare Polymere (wie Polyethylenglykole); für orale Applikation geeignete Zubereitugen können gewünschtenfalls Geschmacks- und Süßstoffe enthalten.

Die Dosierung kann von verschiedenen Faktoren, wie Applikationsweise, Spezies, Alter und/oder individuellem Zustand abhängen. Die tägliche zu verabreichenden Dosen liegen bei etwa 10-1000 mg/Mensch, vorzugsweise bei 100-500 mg/Mensch und können auf einmal oder mehrere Male verteilt eingenommen werden.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden Bernsteinsäurederivate, sowie deren Natrium- und Kaliumsalze, Methyl-, Ethyl- oder Benzylester:

a) 3-Amino-2-phosphono-bernsteinsäure; Fp: 220 °C (Zers.)

b) 3-Dimethylamino-2-phosphono-bernsteinsäure

c) 3-(N-Methyl-N-propylamino)-2-phosphono-bernsteinsäure

d) 3-(1-Pyrrolidino)-2-phosphono-bernsteinsäure

e) 3-(Imidazol-1-yl)-2-phosphono-bernsteinsäure

f) 3-Aminomethyl-2-phosphono-bernsteinsäure; Fp: 103 °C (Zers.)

g) 3-Dimethylaminomethyl-2-phosphono-bernsteinsäure; Fp: 112 °C (Zers.)

h) 3-(N-Methyl-N-pentylamino)methyl-2-phosphonobernsteinsäure; Fp: 110 °C

i) 3-(2-Dimethylamino-ethyl)-2-phosphono-bernsteinsäure

j) 3-[2-(N-Methyl-N-propylamino)ethyl]-2-phosphonobernsteinsäure

k) 2-Phosphono-3-[2-(pyrrolidin-1-yl)ethyl]bernsteinsäure

l) 3-[2-(Imidazol-1-yl)ethyl]-2-phosphono-bernsteinsäure

m) 3-(3-Aminopropyl)-2-phosphono-bernsteinsäure; Fp: 121°C (Zers.)

n) 2-Phosphono-3-[3-(pyrrolidin-1-yl)propyl]bernsteinsäure

o) 3-(4-Aminobutyl)-2-phosphono-bernsteinsäure; Fp: 135 °C (Zers.)

p) 2-Phosphono-3-[4-(pyrrolidin-1-yl)butyl]bernsteinsäure

q) 3-(5-Aminopentyl)-2-phosphono-bernsteinsäure

r) 2-Phosphono-3-[5-(pyrrolidin-1-yl)pentyl]bernsteinsäure

s) 3-[5-(Imidazol-1-yl)pentyl]-2-phosphono-bernsteinsäure

t) 3-(6-Aminohexyl)-2-phosphono-bernsteinsäure

u) 2-Phosphono-3-[6-(pyrrolidin-1-yl)hexyl]bernsteinsäure

v) 3-[6-(Imidazol-1-yl)hexyl]-2-phosphono-bernsteinsäure

w) 2-Phosphono-3-(pyrid-2-yl)bernsteinsäure

x) 2-Phosphono-3-(pyrid-3-yl)bernsteinsäure

y) 2-Phosphono-3-(pyrid-4-yl)bernsteinsäure

z) 3-(Imidazol-2-yl)-2-phosphono-bernsteinsäure

aa) 3-(Imidazol-4-yl)-2-phosphono-bernsteinsäure

ab) 2-Phosphono-3-(pyrrolidin-2-yl)bernsteinsäure

ac) 2-Phosphono-3-(pyrrolidin-3-yl)bernsteinsäure

ad) 2-Phosphono-3-(pyrid-2-ylmethyl)bernsteinsäure

ae) 2-Phosphono-3-(pyrid-3-ylmethyl)bernsteinsäure

af) 2-Phosphono-3-(pyrid-4-ylmethyl)bernsteinsäure

ag) 3-(Imidazol-2-yl-methyl)-2-phosphono-bernsteinsäure

ah) 3-(Imidazol-4-yl-methyl)-2-phosphono-bernsteinsäure

ai) 2-Phosphono-3-(pyrrolidin-2-yl-methyl)bernsteinsäure

aj) 2-Phosphono-3-(pyrrolidin-3-yl-methyl)bernsteinsäure

ak) 2-Phosphono-3-[2-(pyrid-2-yl)ethyl]bernsteinsäure

al) 2-Phosphono-3-[2-(pyrid-3-yl)ethyl]bernsteinsäure

am) 2-Phosphono-3-[2-(pyrid-4-yl)ethyl]bernsteinsäure

an) 3-[2-(Imidazol-2-yl)ethyl]-2-phosphono-bernsteinsäure

ao) 3-[2-(Imidazol-4-yl)ethyl]-2-phosphono-bernsteinsäure

ap) 2-Phosphono-3-[2-(pyrrolidin-2-yl)ethyl]bernsteinsäure

aq) 2-Phosphono-3-[2-(pyrrolidin-3-yl)ethyl]bernsteinsäure

ar) 3-[3-(Imidazol-4-yl)propyl]-2-phosphono-bernsteinsäure

as) 2-Phosphono-3-[4-(pyrrolidin-2-yl)butyl]bernsteinsäure

at) 3-(N-Allyl-N-methylamino)-2-phosphono-bernsteinsäure

au) 3-(N-Methyl-N-propargylamino)-2-phosphono-bernsteinsäure

av) 3-[4-(N-Allyl-N-methylamino)butyl]-2-phosphonobernsteinsäure

aw) 3-[4-(N-Methyl-N-propargylamino)butyl]-2-phosphono-bernsteinsäure

ax) 3-[4-(N-Ethyl-N-isobutylamino)butyl]-2-phosphono-bernsteinsäure

ay) 3-(Azepin-1-yl-methyl)-2-phosphono-bernsteinsäure

az) 2-Phosphono-3- [1- (pyrrolidin-1-yl)ethyl]bernsteinsäure

ba) 2-Phosphono-3-[2-(pyrid-2-yl)propyl]bernsteinsäure

bb) 2-Phosphono-3-[1-methyl-1-(pyrid-3-yl)ehtyl]bernsteinsäure

bc) 3-[3-(Imidazol-1-yl)-2-methyl-propyl]-2-phosphono-bernsteinsäure

bd) 3-(3-Amino-butyl)-2-phosphono-bernsteinsäure

be) 3-[1,1-Dimethyl-3-(N-methyl-N-pentylamino)propyl]-2-phosphono-bernsteinsäure

bf) 3-[3-(Imidazol-4-yl)-2,3-dimethyl-propyl-bernsteinsäure

bg) 3-(2,2-Dimethyl-3-dimethylamino-propyl)-2-phosphono-bernsteinsäure

bh) 3-[2-Methyl-4-(pyrrolidin-2-yl)butyl]-2-phosphono-bernsteinsäure

bi) 3-[2,3-Dimethyl-4-(pyrrolidin-2-yl)butyl]-2-phosphono-bernsteinsäure

bj) 3-(5-Amino-2-methyl-pentyl)-2-phosphono-bernsteinsäure

bk) 2-Phosphono-3-[4-(pyrid-2-yl)-but-2-enyl]bernsteinsäure

bl) 2-Phosphono-3-[4-(pyrid-4-yl)-but-2-inyl]bernsteinsäure

Die nachfolgenden Beispiele zeigen einige der Verfahrensvarianten, die zur Synthese der erfindungsgemäßen Verbindungen verwendet werden können. Sie sollen jedoch nicht eine Einschränkung des Erfindungsgegenstandes darstellen. Die Struktur der Verbindungen wurde durch $^{1}H$-, $^{31}P$- und gegebenenfalls durch $^{13}C$-NMR-Spektroskopie gesichert. Die Reinheit der Substanzen wurde mittels C, H, N, P, gegebenenfalls Na-Analyse sowie dünnschichtchromatographisch bzw. durch Dünnschichtelektrophorese (Cellulose, Oxalat-Puffer von pH = 4.0) bestimmt.

## Beispiel 1

### 2-Diethylphosphono-3-methoxycarbonyl-5-phthaloylimidovaleriansäureethylester

Zu 240 mg (10 mmol) Natriumhydrid in 10 ml absolutem Toluol tropft man unter Kühlung 2.24 g (10 mmol) Phosphonoessigsäuretriethylester. Nach Beendigung der Wasserstoffentwicklung tropft man eine Lösung von 3.26 g (10 mmol) 2-Brom-4-phthaloylimido-buttersäuremethylester (Hoppe Seyler Z. Physiol. Chem. 1967, 1600) in 70 ml absolutem Toluol zu und läßt 24 Stunden bei Zimmertemperatur rühren. Die Lösung wird mit ca. 1 ml etherischer Salzsäure neutralisiert, am Rotationsverdampfer eingeengt, und das verbleibende Öl über 200 g Kieselgel gereinigt (Fließmittel: Aceton/Toluol i.V. 1/1). Man erhält 2.8 g = 60 % eines farblosen öls, dessen Struktur durch NMR-Spektroskopie bestätigt wurde.

## Beispiel 2

### 3-(2-Amino-ethyl)-2-phosphono-bernsteinsäure

1.5 g (3.2 mmol) des in Beispiel 1 beschriebenen Tetraesters werden in 40 ml 6 N Salzsäure 8 Stunden unter Rückfluß erhitzt. Die Lösung wird auf ca. 10 ml eingeengt, der entstandene Niederschlag abgesaugt, das Filtrat vollständig eingeengt, der Rückstand mit 3 ml Wasser verrührt, abgesaugt und das Filtrat wieder eingeengt. Man erhält ein bräunliches Öl, das in 2 ml Wasser gelöst wird und über 25 g Ionenaustauscher (Amberlite IR 120; $H^{+}$-Form) gegeben wird. Die Säule wird mit Wasser eluiert und die Fraktionen mit der gewünschten Substanz eingeengt. Man erhält 0.34 g = 40 % eines weißen, amorphen Pulvers mit einem Fp: 127-130°C unter Zersetzung.

Beispiel 3

2-Diethylphosphono-3-ethoxycarbonyl-7-(imidazol-1-yl)heptancarbonsäureethylester

Zu 48 mg (2 mmol) Natriumhydrid in 2 ml absolutem Toluol tropft man 552 mg (4 mmol) Diethylphosphit und nach weiteren 5 Minuten eine Lösung von 588 mg (2 mmol) 4-(Imidazol-1-yl)butyl-fumarsäurediethylester in 4 ml absolutem Toluol. Nach 20 Stunden neutralisiert man mit etherischer Salzsäure, zieht das Lösungsmittel ab und reinigt den öligen Rückstand über 200 g Kieselgel (Fließmittel: Aceton/Toluol i.v. 1/1). Man erhält 380 mg = 44 % eines gelblichen Öls.

Den als Ausgangsmaterial eingesetzten 4-(Imidazol-I-yl)butyl-fumarsäurediethylester erhält man auf folgende Weise:

Zu 72 mg (3 mmol) Natriumhydrid in 3 ml absolutem Dimethylformamid gibt man 204 mg (3 mmol) Imidazol. Nach 15 Minuten gibt man zu der klaren gelblichen Lösung 921 mg (3 mmol) (4-Brom-butyl)fumarsäurediethylester (Tetrahedron Letters 22, 381 (1981)). Man läßt über Nacht rühren, neutralisiert mit etherischer Salzsäure, engt ein und reinigt das zurückbleibende Öl über 150 g Kieselgel (Fließmittel: Aceton/Toluol i.v. 1/1). Man erhält 750 mg = 41 % der gewünschten Substanz als öl.

Beispiel 4

3-[4-(Imidazol-1-yl)butyl]-2-phosphonobernsteinsäure

432 mg (1 mmol) des in Beispiel 3 beschriebenen Tetraesters werden in 15 ml 6 N Salzsäure 6 Stunden unter Rückfluß erhitzt. Die Lösung wird dann eingeengt, der Rückstand in 2 ml Wasser gelöst und über 20 g Ionenaustauscher (Amberlite IR 120; $H^+$-Form) gegeben. Die Säule wird mit Wasser eluiert und die Fraktionen mit der gewünschten Substanz eingeengt. Man erhält 165 mg = 52 % eines weißen, amorphen Pulvers mit einem Fp: 161-164°C unter Zersetzung.

Beispiel 5

2-Diethylphosphono-3-methoxycarbonyl-4-(pyrrolidin-1-yl)buttersäuremethylester

Zu 1.1 g (3.74 mmol) 2-Diethylphosphono-3-methoxycarbonyl-but-3-ensäuremethylester in 10 ml absolutem Toluol gibt man 265 mg (3.74 mmol) frisch destilliertes Pyrrolidin. Man läßt die Lösung 24 Stunden bei Zimmertemperatur stehen, engt ein und reinigt den Rückstand über 100 g Kieselgel (Fließmittel: Aceton/Toluol i.V. 1/4). Man erhält 490 mg = 38 % der gewünschten Substanz als öl. Das NMR-Spektrum bestätigt die Struktur.

Den als Ausgangsmaterial eingesetzten 2-Diethylphosphono-3-methoxycarbonyl-but-3-ensäuremethylester stellt man auf folgende Weise her:

Zu 7.19 g (30 mmol) 2-Brommethyl-fumarsäuredimethylester (J. Org. Chem. 34, 1228 (1969)) tropft man langsam 5.2 ml (30 mmol) Triethylphosphit. Die Innentemperatur steigt dabei auf 90°C. Man erhitzt dann 1 Stunde auf 150°C, läßt abkühlen und reinigt das Öl über eine Kieselgelsäule (Fließmittel: Aceton/Toluol i.V. 1/4). Man erhält 4.9 g = 54 % der gewünschten Substanz als öl. Die Struktur wurde durch NMR- und Massenspektroskopie bestätigt.

Beispiel 6

2-Phosphono-3-(pyrrolidin-1-yl-methyl)bernsteinsäure

3.65 g (10 mmol) des in Beispiel 5 beschriebenen Tetraesters werden mit 50 ml 6 N Salzsäure 6 Stunden unter Rückfluß erhitzt. Die Lösung wird dann eingeengt, der Rückstand in 20 ml Wasser gelöst und über einen Ionenaustauscher (Amberlite IR 120; $H^+$-Form) gereinigt. Die Fraktionen mit der gewünschten Substanz werden eingeengt und getrocknet. Man erhält 2.14 g = 74 % eines weißen Pulvers mit 0.5 mol Kristallwasser; Fp: 122-l24°C unter Zersetzung.

Beispiel 7

2-Diethylphosphono-4-(imidazol-1-yl)-3-methoxycarbonyl-buttersäuremethylester

Zu 75 mg (3 mmol) Natriumhydrid in 10 ml absolutem Tetrahydrofuran tropft man 205 mg (3 mmol) Imidazol in 10 ml absolutem Tetrahydrofuran. Nach Beendigung der Wasserstoffentwicklung gibt man 1.18 g (4 mmol) 2-Diethylphosphono-3-methoxycarbonyl-but-3-ensäuremethylester (s. Beispiel 5) in 20 ml absolutem Tetrahydrofuran zu und läßt 72 Stunden rühren. Man engt die Mischung ein, gibt 20 ml Wasser zu, stellt mit 2 N Salzsäure auf pH = 6 ein und

extrahiert mehrmals mit Methylenchlorid. Die vereinigten organischen Phasen werden getrocknet und eingeengt. Der Rückstand wird über 100 g Kieselgel (Fließmittel: Aceton/Toluol i.V. 3/1) gereinigt. Man erhält 610 mg = 61 % der gewünschten Substanz als Öl. Das NMR-Spektrum bestätigt die Struktur.

Beispiel 8

3-(Imidazol-1-ylmethyl)-2-phosphono-bernsteinsäure

1.08 g (3 mmol) des in Beispiel 7 beschriebenen Tetraesters werden mit 30 ml 6 N Salzsäure 6 Stunden unter Rückfluß erhitzt. Man engt dann die Lösung ein, nimmt den Rückstand in wenig Wasser auf, bringt die Lösung mit 2 N Natronlauge auf einen pH = 5, versetzt sie mit dem dreifachen Volumen Methanol und läßt im Kühlschrank stehen. Der gebildete Niederschlag wird abgesaugt, mit Methanol gewaschen und getrocknet. Man erhält 487 mg = 47 % eines weißen Pulvers als Dinatriumsalz mit 2 mol Kristallwasser; Fp: 135-137°C unter Zersetzung.

**Pharmakologischer Vergleichsversuch**

Beispiel 9

Osteoclasten-Assay

Material und Methode:

Die versuchsdurchführung erfolgte nach der Methode von P. Collin, H. Günther und H. Fleisch (Endocrinol. 131, 1181-87, 1992) unter Verwendung von frisch isolierten Osteoclasten.

Besonderheiten:

Die im Medium 199 (Gibco AG, Basel, Schweiz) bei pH 7.36 suspendierte osteoclastenpräparation wird 5 Minuten vor und 25 Minuten währen der Adherierung auf Waldentin, sowie während der 24 Stunden Assay-Zeit (in MEM Earle's) mit $10^{-8}$ M Substanz behandelt.

Die Berechnung der Wirkung (% Resorptionshemmung) erfolgte in diesem Assay nach folgender Formel:

$$\% \text{ Resorptionshemmung} = \frac{\text{Anzahl "pits" Behandelt}}{\text{Anzahl "pits" Unbehandelt}} \times 100$$

| Beispiel Nr. | Systematischer Name | Resorptionshemmmung |
|---|---|---|
| 2 | 3-(2-Amino-ethyl)-2-phosphono-bernsteinsäure | 80% |
| 6 | 2-Phosphono-3-(pyrrolidin-1yl-methyl)-bernsteinsäure | 71% |
| 8 | 3-(Imidazol-1yl-methyl)-2-phosphono-bernsteinsäure | 73% |
| g) | 3-Dimethylamino-methyl-2-phosphono-bernsteinsäure | 60% |
| a) | 3-Amino-2-phosphono-bernsteinsäure | 59% |
| h) | 3-(N-Methyl-N-pentylamino)-methyl-2-phosphono-bernsteinsäure | 51% |

**Patentansprüche**

**1.** Phosphonobernsteinsäure-Derivate der Formel I

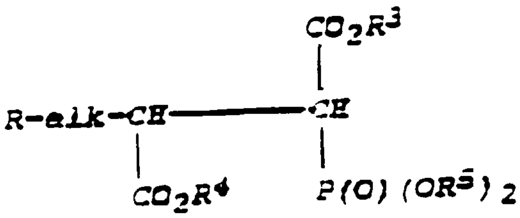

(I)

in der

R eine gegebenenfalls substituierte Aminogruppe der allgemeinen Formel $-NR_1R_2$, wobei $R_1$ und $R_2$ unabhängig voneinander jeweils Wasserstoff, $C_1$-$C_6$-Alkyl, $C_3$-$C_6$-Akenyl oder $C_3$-$C_6$-Alkinyl bedeutet, oder R einen heterocyclischen Ring darstellt, der aus der Gruppe ausgewählt ist, die den Aziridin-, Azetidin-, Pyrrolidin-, Piperidin-, Azepin-, Morpholin-, Thiomorpholin-, Imidazolin-, Pyridin-, Pyrimidin-, Pyrazin- und Imidazolring enthölt, der gegebenenfalls ein- oder zweifach durch $C_1$-$C_6$-Alkyl, Chlor oder Brom substituiert sein kann,

alk einen Valenzstrich, eine Methylen-, eine gesättigte oder ungesättigte, geradkettige oder verzweigte Alkylenkette mit 2-6 Kohlenstoffatomen bedeutet, und

$R_3$, $R_4$, $R_5$ jeweils unabhängig voneinander Wasserstoff, $C_1$-$C_6$-Alkyl oder Benzyl bedeuten,

sowie deren pharmakologisch unbedenkliche Salze und Enantiomere wobei für den Fall, daß $R^3=R^4=R^5=CH_3$ und alk einen Valenzstrich bedeutet, R nicht die Dimethylaminogruppe sein darf.

**2.** Verfahren zur Herstellung von Phosphonobernsteinsäure-Derivaten der Formel I

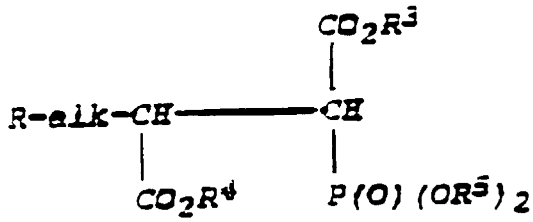

(I)

in der

R eine gegebenenfalls substituierte Aminogruppe der allgemeinen Form $NR_1R_2$, wobei $R_1$ und $R_2$ unabhängig voneinander jeweils Wasserstoff, $C_1$-$C_6$-Alkyl, $C_3$-$C_6$-Akenyl oder $C_3$-$C_6$-Alkinyl bedeutet, oder R einen heterocyclischen Ring darstellt, der aus der Gruppe ausgewählt ist, die den Aziridin-, Azetidin-, Pyrrolidin-, Piperidin-, Azepin-, Morpholin-, Thiomorpholin-, Imidazolin-, Pyridin-, Pyrimidin-, Pyrazin- und Imidazolring enthölt, der gegebenenfalls ein- oder zweifach durch $C_1$-$C_6$-Alkyl, Chlor oder Brom substituiert sein kann,

alk einen Valenzstrich, eine Methylen-, eine gesättigte oder ungesättigte, geradkettige oder verzweigte Alkylenkette mit 2-6 Kohlenstoffatomen bedeutet, und

$R^3$, $R^4$, $R^5$ jeweils unabhängig voneinander Wasserstoff, $C_1$-$C_6$-Alkyl oder Benzyl bedeuten,

sowie deren pharmakologisch unbedenkliche Salze und Enantiomere wobei für den Fall, daß $R^3=R^4=R^5=CH_3$ und alk einen Valenzstrich bedeutet, R nicht die Dimethylaminogruppe sein darf,

dadurch gekennzeichnet, daß man in an sich bekannter Weise

a) Carbonsäurederivate der allgemeinen Formel II;

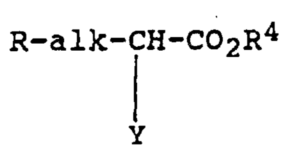

(II),

in der R, alk und $R^4$ die oben angegebenen Bedeutungen haben und Y eine Abgangsgruppe, wie z.B. Hal oder $O-SO_2-Z$ bedeuten, wobei Hal Chlorid, Bromid oder Jodid und Z Methyl, Phenyl, p-Methylphenyl oder p-Nitrophenyl sein sollen, mit einem Phosphonoessigsäureester der allgemeinen Formel III,

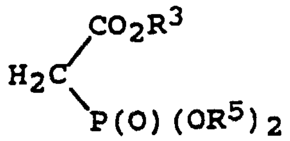

(III),

in der $R^3$ und $R^5$ die oben angegebene Bedeutung besitzen, umsetzt, wobei für den Fall, daß R eine primäre oder sekundäre Aminogruppe bedeutet, diese in geschützter Form etwa als Acylamino- oder Phthaloylimidogruppe vorliegen muß, und gegebenenfalls die entstandenen Ester teilweise oder vollständig zu den entsprechenden Säuren der allgemeinen Formel I verseift, oder

b) Verbindungen der allgemeinen Formel IV,

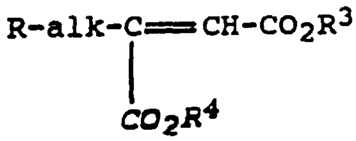

(IV),

in der R, alk, $R^3$ und $R^4$ die oben angegebenen Bedeutungen haben, mit einem Dialkylphosphit der allgemeinen Formel V,

$$H\text{-}P(O)(OR^5)_2 \qquad (V)$$

in der $R^5$ die oben angegebener Bedeutungen hat, umsetzt und gegebenenfalls die entstandenen Ester teilweise oder vollständig zu den entsprechenden Säuren der allgemeinen Formel I verseift, oder

c) eine Verbindung der allgemeinen Formel VI oder VII,

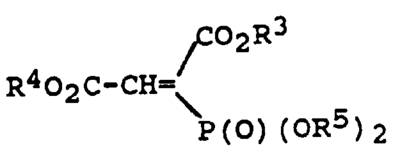

,

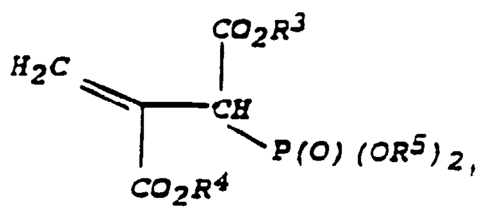

VI

VII

in der $R^3$, $R^4$ und $R^5$ die oben angegebenen Bedeutungen besitzen, in an sich bekannter Weise mit einer Verbindung der allgemeinen Formel VIII,

$$R\text{-}alk\text{-}M \qquad (VIII)$$

in der R die oben genannten Bedeutungen besitzt, und M Wasserstoff oder ein Alkali- bzw. Erdalkalimetall bedeutet, zur Reaktion bringt und gegebenenfalls die entstandenen Ester teilweise oder vollständig zu den entsprechenden Säuren der allgemeinen Formel I verseift und gewünschtenfalls in pharmakologisch verträgliche Salze überführt.

**3.** Arzneimittel, enthaltend mindestens eine Verbindung gemäß Anspruch 1 neben üblichen Träger- und Hilfsstoffen.

**4.** Verwendung von Verbindungen gemäß Anspruch 1 zur Herstellung von Arzneimitteln zur Behandlung von calcium-Stoffwechselstörungen.

## Claims

**1.** Phosphonosuccinic acid derivatives of the formula I

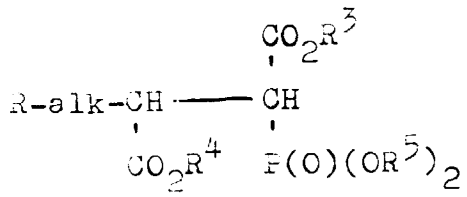

(I)

in which R signifies a possibly substituted amino group of the general formula $-NR^1R^2$, whereby $R^1$ and $R^2$, independently of one another, each signify hydrogen, $C_1$-$C_6$-alkyl, $C_3$-$C_6$-alkenyl or $C_3$-$C_6$-alkynyl or R represents a heterocyclic ring which is selected from the group which contains the aziridine, azetidine, pyrrolidine, piperidine, azepine, morpholine, thiomorpholine, imidazoline, pyridine, pyrimidine, pyrazine and imidazole ring, which can possibly be substituted once or twice by $C_1$-$C_6$-alkyl, chlorine or bromine, alk signifies a valency bond, a methylene, a saturated or unsaturated, straight-chained or branch alkylene chain with 2-6 carbon atoms and $R^3$, $R^4$, $R^5$, in each case independently of one another, signify hydrogen, $C_1$-$C_6$-alkyl or benzyl, as well as their pharmacologically acceptable salts and enantiomers, whereby, for the case that $R^3 = R^4 = R^5 = CH_3$ and alk signifies a valency bond, R cannot be the dimethylamino group.

**2.** Process for the preparation of phosphonosuccinic acid derivatives of the formula I

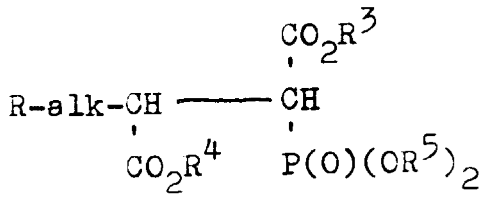

(I)

in which R signifies a possibly substituted amino group of the general formula $-NR^1R^2$, whereby $R^1$ and $R^2$, independently of one another, each signify hydrogen, $C_1$-$C_6$-alkyl, $C_3$-$C_6$-alkenyl or $C_3$-$C_6$-alkynyl or R represents a heterocyclic ring which is selected from the group which contains aziridine, azetidine, pyrrolidine, piperidine, azepine, morpholine, thiomorpholine, imidazoline, pyridine, pyrimidine, pyrazine and imidazole ring which can possibly be substituted once or twice by $C_1$-$C_6$-alkyl, chlorine or bromine, alk signifies a valency bond, a methylene, a saturated or unsaturated, straight-chained or branched alkylene chain with 2 - 6 carbon atoms and $R^3$, $R^4$, $R^5$, in each case independently of one another, signify hydrogen, $C_1$-$C_6$-alkyl or benzyl, as well as of their pharmacologically acceptable salts and enantiomers, whereby, for the case that $R^3 = R^4 = R^5 = CH_3$ and alk signifies a valency bond, R cannot be the dimethylamino group, characterised in that, in per se known manner, one

a) reacts carboxylic acid derivatives of the general formula II

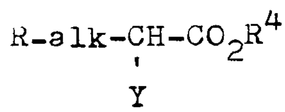

(II)

in which R, alk and $R^4$ have the above-given meanings and Y is a group which can be removed, such as e.g. Hal or $O$-$SO_2$-Z, whereby Hal is to be chloride, bromide or iodide and Z methyl, phenyl, p-methyl-phenyl or p-nitrophenyl, with a phosphonoacetic acid ester of the general formula III

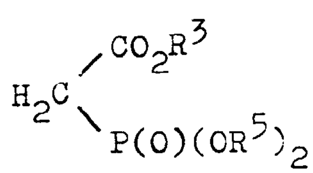

(III)

in which $R^3$ and $R^5$ possess the above-given meaning, whereby, for the case that R signifies a primary or secondary amino group, this must be present in protected form, perhaps as acylamino or phthaloylimido group, end possibly partly or completely saponifies the resultant esters to the corresponding acids of the general formula I, or

b) reacts compounds of the general formula IV

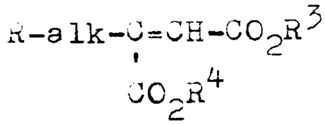

(IV)

in which R, alk, $R^3$ and $R^4$ have the above-given meanings, with a dialkyl phosphite of the general formula V

$$H\text{-}P(O)(OR^5)_2 \qquad (V)$$

in which $R^5$ has the above-given meaning, and possibly partly or completely saponifies the resultant esters to the corresponding acids of the general formula I, or

c) brings a compound of the general formula VI or VII

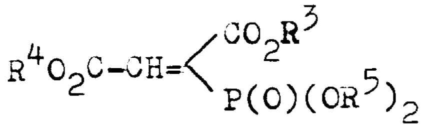

,

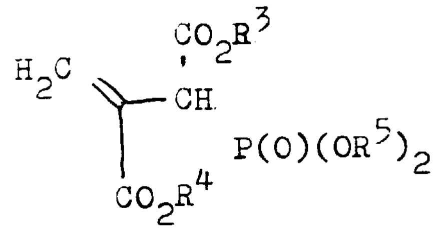

VI VII

in which $R^3$, $R^4$ and $R^5$ possess the above-given meanings, to reaction in per se known manner with a compound of the general formula VIII

$$R\text{-alk-}M \qquad (VIII)$$

in which R possesses the above-given meanings and M signifies hydrogen or an alkali metal or alkaline earth metal, and possibly partly or completely saponifies the resultant esters to the corresponding acids of the general formula I and, if desired, converts into pharmacologically acceptable salts.

**3.** Medicaments containing at least one compound according to claim 1, besides usual carrier and adjuvant materials.

**4.** Use of compounds according to claim 1 for the production of medicaments for the treatment of calcium metabolism disturbances.

**Revendications**

**1.** Dérivés de l'acide phosphonosuccinique de formule I

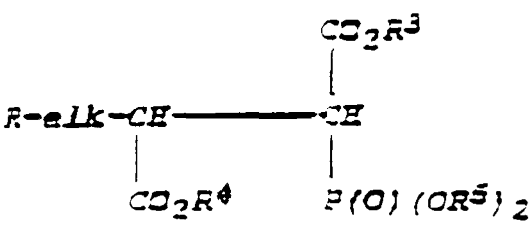

(I)

dans laquelle

R représente un groupe amino éventuellement substitué de formule $-NR_1R_2$, où $R_1$ et $R_2$ représentent chacun indépendamment un atome d'hydrogène, un groupe alkyle en $C_1$-$C_6$, alcényle en $C_3$-$C_6$ ou alcinyle en $C_3$-$C_6$ ou R représente un hétérocycle choisi dans le groupe contenant les cycles aziridine, azétidine, pyrrolidine, pipéridine, azépine, morpholine, thiomorpholine, imidazoline, pyridine, pyrimidine, pyrazine et imidazoline, qui peuvent porter éventuellement un ou deux substituants alkyle en $C_1$-$C_6$, chlore ou brome,

alk représente une valence libre, une chaîne méthylénique, une chaîne alkylène saturée ou insaturée, linéaire ou ramifiée ayant 2-6 atomes de carbone, et

$R_3$, $R_4$, $R_5$ représentent chacun indépendamment un atome d'hydrogène, un groupe alkyle en $C_1$-$C_6$ ou un groupe benzyle,

ainsi que leurs sels et énantiomères pharmacologiquement acceptables, étant entendu que dans le cas où $R^3=R^4=R^5=CH_3$ et alk représente une valence libre, R ne doit pas représenter le groupe diméthylamino.

**2.** Procédé de préparation de dérivés de l'acide phosphonosuccinique de formule I

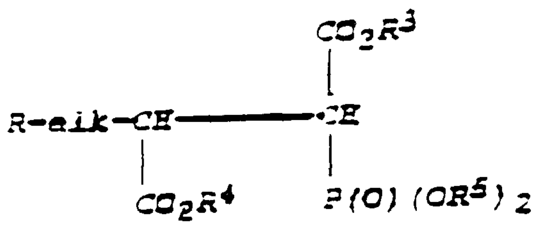

(I)

dans laquelle

R représente un groupe amino éventuellement substitué de formule $-NR_1R_2$, où $R_1$ et $R_2$ représentent chacun indépendamment un atome d'hydrogène, un groupe alkyle en $C_1$-$C_6$, alcényle en $C_3$-$C_6$ ou alcinyle en $C_3$-$C_6$ ou R représente un hétérocycle choisi dans le groupe contenant les cycles aziridine, azétidine, pyrrolidine, pipéridine, azépine, morpholine, thiomorpholine, imidazoline, pyridine, pyrimidine, pyrazine et imidazoline, qui peuvent porter éventuellement un ou deux substituants alkyle en $C_1$-$C_6$, chlore ou brome,

alk représente une valence libre, une chaîne méthylénique, une chaîne alkylène saturée ou insaturée, linéaire ou ramifiée ayant 2-6 atomes de carbone, et

$R^3$, $R^4$, $R^5$ représentent chacun indépendamment un atome d'hydrogène, un groupe alkyle en $C_1$-$C_6$ ou un groupe benzyle,

ainsi que leurs sels et énantiomères pharmacologiquement acceptables, étant entendu que dans le cas où $R^3=R^4=R^5=CH_3$ et alk représente une valence libre, R ne doit pas représenter le groupe diméthylamino,

caractérisé par le fait que de manière connue en soi,

a) on fait réagir des dérivés d'acides carboxyliques de formule générale II

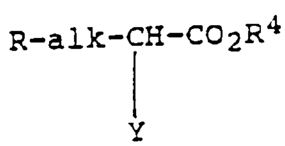

(II).

dans laquelle R, alk et $R^4$ ont les significations indiquées ci-dessus et Y représente un groupe partant, comme par exemple Hal ou $O-SO_2-Z$, où Hal doit être un chlorure, bromure ou iodure et Z, un groupe méthyle, phényle, p-méthylphényle ou p-nitrophényle, avec un phosphonoacétate de formule générale III,

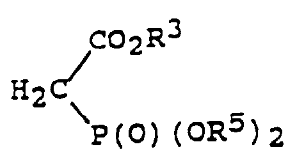

(III),

dans laquelle $R^3$ et $R^5$ ont les significations indiquées ci-dessus, étant entendu que dans le cas où R représente un groupe amino primaire ou secondaire, ce dernier doit se trouver sous forme protégée, par exemple sous

forme de groupe acylamino ou phtaloylimido, et éventuellement, on saponifie partiellement ou totalement l'ester formé pour obtenir l'acide correspondant, de formule générale I, ou

b) on fait réagir des composés de formule générale IV

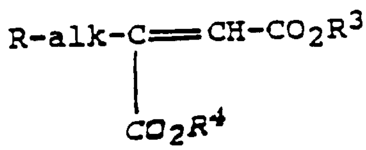

(IV),

dans laquelle R, alk, $R^3$ et $R^4$ ont les significations indiquées ci-dessus, avec un dialkylphosphite de formule générale V

$$H\text{-}P(O)(OR^5)_2 \qquad (V)$$

dans laquelle $R^5$ a la signification indiquée plus haut, et éventuellement, on saponifie l'ester formé, partiellement ou totalement, en l'acide correspondant, de formule générale I, ou

c) on fait réagir un composé de formule générale VI ou VII

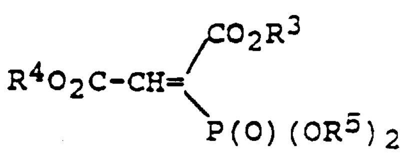

,

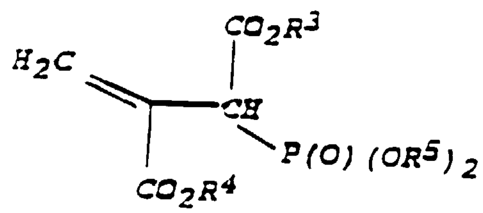

VI

VII

dans laquelle $R^3$, $R^4$ et $R^5$ ont les significations indiquées plus haut, de manière connue en soi avec un composé de formule générale VIII,

$$R\text{-}alk\text{-}M \qquad (VIII)$$

dans laquelle R a la signification indiquée plus haut et M représente un atome d'hydrogène ou un métal alcalin ou alcalinoterreux et éventuellement, on saponifie partiellement ou totalement l'ester formé en l'acide correspondant, de formule générale I et on transforme celui-ci en un sel pharmacologiquement acceptable.

**3.** Médicament, contenant au moins un composé selon la revendication 1 en plus de véhicules et adjuvants usuels.

**4.** Utilisation de composés selon la revendication pour la préparation de médicaments destinés au traitement de troubles du métabolisme calcique.